# EUROPEAN PATENT APPLICATION

(11) **EP 4 092 104 A1**
(43) Date of publication of application: **23.11.2022**
(21) Application number: 21741240.2
(22) Date of filing: 13.01.2021
(51) Int. Cl.: C12N 5/02

(54) **MEDIUM HAVING REDUCED OSMOTIC PRESSURE**

(30) Priority: 14.01.2020 JP 2020003960
(71) Applicant: AJINOMOTO CO., INC., Chuo-ku Tokyo 104-8315 (JP)
(72) Inventor: OGAWA, Shimpei, Kawasaki-shi, Kanagawa 210-8681 (JP); HIGUCHI, Takuya, Kawasaki-shi, Kanagawa 210-8681 (JP); FUROMITSU, Shumpei, Kawasaki-shi, Kanagawa 210-8681 (JP); OHYA, Yusuke, Kawasaki-shi, Kanagawa 210-8681 (JP); NISHIYAMA, Megumi, Kawasaki-shi, Kanagawa 210-8681 (JP); KONISHI, Atsushi, Kawasaki-shi, Kanagawa 210-8681 (JP)
(74) Representative: Hoffmann Eitle
(86) International application number: PCT/JP2021/000747
(87) International publication number: WO 2021/145322

(57) **Abstract**

A medium with a reduced osmotic pressure containing a basal medium, in which the osmotic pressure is adjusted to 150 to 250 mOsm/kg, is provided by the present invention.

## Description

### [Technical Field]

The present invention relates to a medium composition with a reduced osmotic pressure and a cell culture method using same.

### [Background Art]

Regenerative medicine technology using cells has been receiving high attention in recent years as one of the means capable of treating various diseases and damages that were difficult to treat before. Since regenerative medicine requires a large amount of cells, the development of a method for efficiently culturing cells has been actively conducted. For example, Patent Literature 1 reports a method for culturing stem cells, including treating stem cells with a ROCK inhibitor in a medium.

Stable culture and proliferation of cells essentially requires operations such as regular medium exchange, passaging, and the like. Since such operation involves repetition of complicated processes, there arise problems that the burden on the operator is heavy and that the cell proliferation efficiency and the like are affected by the skill of the operator. For this reason, cell culture devices that monitor the culture environment, such as pH and gas concentration of the medium, and automatically optimize them as necessary (sometimes referred to as "bioreactor" or the like in the present specification), have been developed and utilized in recent years. One example of such bioreactor is, for example, "ambr (registered trade mark)" cell culture device from Sartorius.

### [Citation List]

### [Patent Literature]

[PTL 1]
JP-A- 2008-099662

### [Summary of Invention]

### [Technical Problem]

The use of bioreactors greatly improves the burden on operators and problems caused by operators during cell culture. On the other hand, however, it is difficult to say at this point that sufficient studies have been made to determine what conditions should be employed to optimize the efficiency of cell growth when using a bioreactor.

Therefore, the purpose of the present invention is to provide, in cell culture using a bioreactor, a means for increasing the efficiency of cell proliferation.

### [Solution to Problem]

The present inventors have conducted intensive studies of the above-mentioned problems and found that the osmotic pressure of a culture medium is one of the factors that affect the cell proliferation efficiency in cell culture. In addition, they have found that this fact is extremely important in cell culture using a bioreactor. That is, in cell culture, the pH of the medium decreases due to the vital activity of the cells over time, but the bioreactor detects a decrease in the pH in the medium and automatically adds a pH adjuster (e.g., sodium hydrogencarbonate) to the medium. As a result, the pH of the medium is maintained near neutral. On the other hand, the osmotic pressure of the medium also increases as the amount of the pH adjuster added increases. When the osmotic pressure of the medium exceeds a certain level, the cell proliferation efficiency begins to be adversely affected. The present inventors have found that this inhibits high density culture in cell culture using a bioreactor. Based on such findings, the present inventors have conducted further studies and completed the present invention.

Accordingly, the present invention provides the following.
[1] A medium with a reduced osmotic pressure comprising a basal medium, wherein the osmotic pressure is adjusted to 150 to 250 mOsm/kg.
[2] The medium of [1], wherein the osmotic pressure of the medium is 170 to 230 mOsm/kg.
[3] The medium of [1] or [2], wherein the osmotic pressure is adjusted by reducing an amount of sodium chloride among the components constituting the basal medium.
[4] A method for culturing a cell, comprising a step of exchanging a part or whole amount of a medium being used with the medium of any of [1] to [3] so that the osmotic pressure of the medium being used is 220 to 310 mOsm/kg.
[5] The method of [4], wherein the cell culture is suspension culture.

### [Advantageous Effects of Invention]

According to the present invention, the osmotic pressure of a medium, which increases over time to maintain pH in the process of cell culture, can be maintained in an appropriate range. Therefore, by applying the present invention, for example, more preferable high density culture can be performed as compared with conventional embodiments of automatic cell culture using a bioreactor.

### [Brief Description of Drawings]

[Fig. 1]

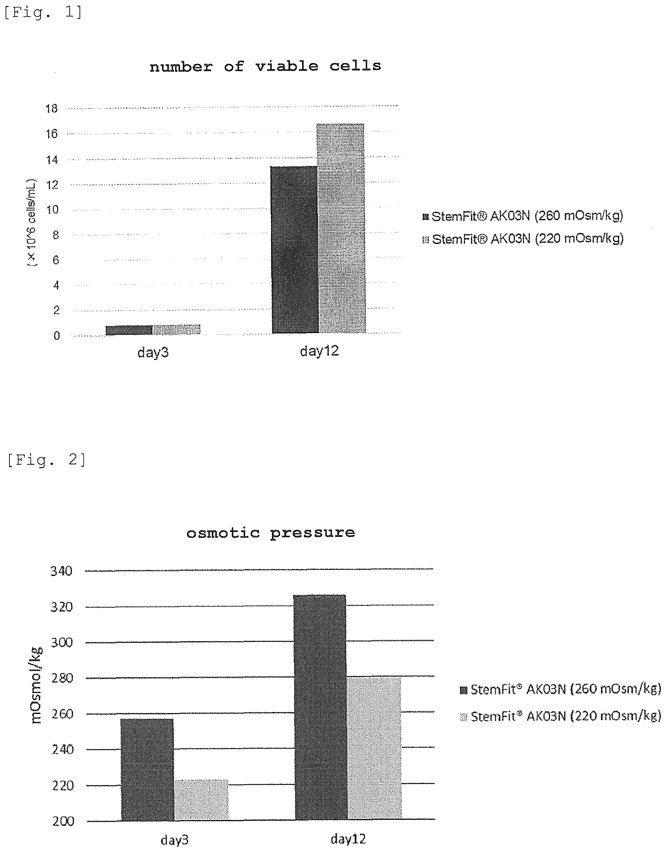

   Fig. 1 shows the influence of the osmotic pressure on the proliferation of iPS cells in Example 1 (n=1).
[Fig. 2]
   Fig. 2 shows the osmotic pressure at the time points of day 3 and day 12 of the medium in which iPS cells were cultured in Example 1.
[Fig. 3]

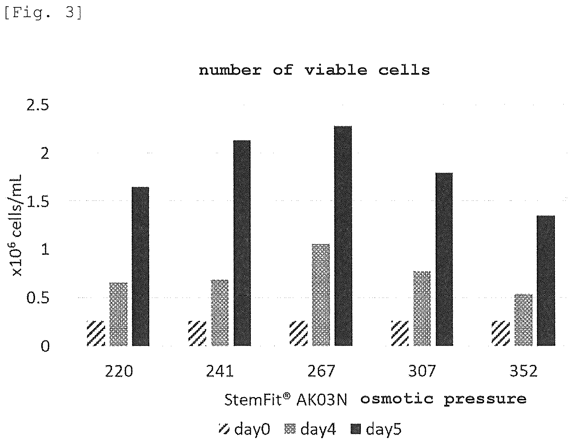

   Fig. 3 shows the influence of the osmotic pressure on the proliferation of iPS cells in Example 2 (n=1).

### [Description of Embodiments]

The present invention is explained in detail in the following.

### Definition

In the present specification, the "suspension culture" refers to a cell culture method performed in a state where cells do not adhere to the culture container. In the present invention, the suspension culture may or may not be accompanied by pressure from the outside or vibration on the liquid medium, or shaking or rotation operation in the liquid medium.

In the present specification, the "basal medium" refers to a medium containing a carbon source, a nitrogen source, an inorganic salt, and the like that are essential for culturing cells.

### 1. medium with reduced osmotic pressure

The present invention provides a medium with a reduced osmotic pressure containing a basal medium, in which the osmotic pressure is adjusted to 150 to 250 mOsm/kg (hereinafter sometimes referred to as "the medium of the present invention").

The medium of the present invention contains a basal medium. The basal medium that can be used as a constituent component of the medium of the present invention is not particularly limited and may be appropriately selected according to the type of cells to be cultured. The basal medium may be prepared by a method known per se, or a commercially available product may also be used.

Examples of the medium that may be contained include Dulbecco's modified Eagle medium (DMEM), Ham's Nutrient Mixture F12, DMEM/F12 medium, McCoy's 5A medium, Minimum Essential medium (MEM), Eagle's Minimum Essential medium (EMEM), alpha Modified Eagle's Minimum Essential medium (αMEM), Roswell Park Memorial Institute (RPMI) 1640 medium, Iscove's Modified Dulbecco's medium (IMDM), MCDB131 medium, William's medium E, Fischer's medium, and the like.

Examples of the basal medium to be used when the medium of the present invention is prepared particularly for culturing stem cells include STEMPRO (registered trade mark) hESC SFM medium (Life Technologies), mTeSR1 medium (STEMCELL Technologies), TeSR2 medium (STEMCELL Technologies), TeSR-E8 medium (STEMCELL Technologies), Essential 8 medium (Life Technologies), HEScGRO (trade mark) Serum-Free medium for hES cells (Millipore), PluriSTEM (trade mark) Human ES/iPS medium (EMD Millipore), NutriStem (registered trade mark) hESC XF medium (Biological Industries Israel Beit-Haemek), NutriStem (trade mark) XF/FF Culture medium (Stemgent), AF NutriStem (registered trade mark) hESC XF medium (Biological Industries Israel Beit-Haemek), S-medium (DS pharma biomedical), StemFit (registered trade mark) AK03N medium (Ajinomoto Co., Inc.), hESF9 medium, hESF-FX medium, CDM medium, DEF-CS 500 Xeno-Free 3D Spheroid Culture medium (Cellartis), StemFlex medium (Thermo Fisher Scientific), and the like.

Also, in addition to the basal medium, components preferable for cell proliferation can also be further added to the medium of the present invention. Examples of such component include sugars such as glucose, fructose, sucrose, maltose, and the like; amino acids such as asparagine, aspartic acid, glutamine, glutamic acid, and the like; proteins such as albumin, transferrin, and the like; peptides such as glycylglycylglycine, soybean peptide, and the like; serum; vitamins such as choline, vitamin A, vitamin Bs (thiamine, riboflavin, pyridoxine, cyanocobalamin, biotin, folic acid, pantothenic acid, nicotine amide etc.), vitamin C, vitamin E, and the like; fatty acids such as oleic acid, arachidonic acid, linoleic acid, and the like; lipids such as cholesterol and the like; inorganic salts such as sodium chloride, potassium chloride, calcium chloride, magnesium sulfate, sodium dihydrogen phosphate, and the like; trace elements such as zinc, copper, selenium, and the like; buffering agents such as N,N-bis(2-hydroxyethyl)-2-aminoethanesulfonic acid (BES), 4-(2-hydroxyethyl)-1-piperazineethanesulfonic acid (HEPES), N-[tris(hydroxymethyl)methyl]glycine (Tricine), and the like; antibiotics such as amphotericin B, kanamycin, gentamicin, streptomycin, penicillin, and the like; cell adhesion factors and extracellular matrix components such as Type I collagen, Type II collagen, fibronectin, laminin, poly-L-lysine, poly-D-lysine, and the like; cytokines and growth factors such as interleukin, fibroblast growth factor (FGF), hepatocyte growth factor (HGF), transforming growth factor (TGF)-α, transforming growth factor (TGF)-β, vascular endothelium growth factor (VEGF), activin A, and the like; hormones such as dexamethasone, hydrocortisone, estra diol, progesterone, glucagon, insulin, and the like; and the like. Appropriate components can be selected and used according to the type of the cells to be cultured.

In one preferred embodiment, D-glucose and five kinds of amino acids (tryptophan, serine, cysteine (or cystine), methionine, arginine) may be further added to the medium of the present invention. The amount of these components to be added can be appropriately set according to the purpose of the culture, various culture conditions (e.g., cell density, frequency of medium exchange), and the like, by referring to the corresponding description in "2. cell culture method" below.

When the medium of the present invention is applied to stem cells, in one preferred embodiment, bFGF and/or choline may be further added to the medium of the present invention.

The medium of the present invention is characterized in that the osmotic pressure is reduced as compared with general media. The upper limit of the osmotic pressure of the medium of the present invention may be generally 250 mOsm/kg, preferably 240 mOsm/kg, more preferably 235 mOsm/kg, further preferably 230 mOsm/kg, particularly preferably 220 mOsm/kg. The lower limit of the osmotic pressure may be generally 150 mOsm/kg, preferably 160 mOsm/kg, more preferably 165 mOsm/kg, further preferably 170 mOsm/kg, particularly preferably 180 mOsm/kg. In one embodiment, the osmotic pressure of the medium of the present invention can be set to generally 150 to 250 mOsm/kg, preferably 160 to 240 mOsm/kg, more preferably 165 to 235 mOsm/kg, further preferably 170 to 230 mOsm/kg, particularly preferably 180 to 220 mOsm/kg.

To reduce the osmotic pressure of the medium, for example, a method of reducing the amount of a medium component that contributes to an increase in the osmotic pressure can be mentioned. For example, the osmotic pressure may be reduced to a desired level by appropriately decreasing the amount of components such as sodium chloride, etc. in the basal medium. Alternatively, the osmotic pressure can also be easily adjusted by diluting the basal medium with an appropriate amount of water. The osmotic pressure of the medium may be measured by a method known per se.

The cell type to which the medium of the present invention can be applied is not particularly limited. Examples of such cell type include germ cells such as spermatozoon, ovum, and the like, somatic cells constituting the living body, stem cells (pluripotent stem cell, etc.), precursor cells, cancer cells separated from the living body, cells (cell lines) that are separated from the living body, acquire immortalizing ability, and are stably maintained ex-vivo, cells that are separated from the living body and subjected to artificial gene modification, cells that are separated from the living body and subjected to artificial nuclear exchange, and the like. Examples of the somatic cell constituting the living body include, but are not limited to, fibroblast, bone marrow cell, B lymphocyte, T lymphocyte, neutrophil, erythrocyte, platelet, macrophage, monocyte, osteocyte, pericyte, dendritic cell, keratinocyte, adipocyte, mesenchymal cell, epithelial cell, epidermis cell, endothelial cell, vascular endothelial cell, hepatocyte, chondrocyte, cumulus cell, neuronal cells, glial cell, neuron, oligodendrocyte, micro glia, astrocyte, heart cell, esophageal cell, muscle cells (e.g., smooth myocyte or skeleton myocyte), pancreas beta cell, melanocyte, hematopoietic progenitor cell (e.g., CD34 positive cell derived from cord blood), mononuclear cell, and the like. The somatic cell includes, for example, cells taken from any tissue such as skin, kidney, spleen, adrenal gland liver, lung, ovary, pancreas, uterus, stomach, colon, small intestine, large intestine, bladder, prostate, testis, thymus, muscle, connective tissue, bone, cartilage, vascular tissue, blood (including cord blood), bone marrow, heart, eye, brain, neural tissue, and the like.

Stem cell is a cell that has the ability to replicate itself and the ability to differentiate into other multilineage cells. Examples thereof include, but are not limited to, embryonic stem cell (ES cell), embryonal carcinoma cell, embryonic germ cell, induced pluripotent stem cell (iPS cell), neural stem cell, hematopoietic stem cell, mesenchymal stem cell, hepatic stem cell, pancreatic stem cell, muscle stem cell, germ stem cell, intestinal stem cell, cancer stem cell, hair follicle stem cell, and the like.

A cell line is a cell that has acquired infinite proliferation potency through artificial manipulation outside the body. A cell line is a cell that has acquired infinite proliferation potency through artificial manipulation outside the body. Examples thereof include, but are not limited to, CHO (Chinese hamster ovary cell line), HCT116, Huh7, HEK293 (human fetal kidney cell), HeLa (human uterine cancer cell line), HepG2 (human liver cancer cell line), UT7/TPO (human leukemia cell line), MDCK, MDBK, BHK, C-33A, HT-29, AE-1, 3D9, Ns0/1, Jurkat, NIH3T3, PC12, S2, Sf9, Sf21, High Five (registered trade mark), Vero, and the like.

In one preferred embodiment, the cell is a stem cell, more preferably an iPS cell.

The medium of the present invention may be provided in a liquid state, or in a state of being concentrated more than the concentration at the time of use, or in a solid state such as freeze-dried powder and the like to be diluted with a solvent such as water and the like when in use, or dissolved or dispersed in a solvent such as water and the like before use.

### 2. cell culture method

The present invention also provides a method for culturing a cell, including a step of exchanging a part or whole amount of a medium being used with the medium of the present invention so that the osmotic pressure of the medium being used is 220 to 310 mOsm/kg (hereinafter sometimes referred to as "the method of the present invention").

The frequency of medium exchange in cell culture is generally determined in comprehensive consideration of various conditions such as cell density, culture method (adhesion culture/suspension culture), type of cell to be cultured, medium composition, culture conditions (temperature, gas concentration), amount of medium to be exchanged (total amount/partial amount), cost of medium, lifestyle of workers, and the like. It is generally once every two to three days, once a day, or multiple times (e.g., twice) a day. The medium exchange can also be performed at such frequency in the method of the present invention.

On the other hand, taking note of one aspect of the present invention that the osmotic pressure of the medium is maintained within a range preferred for cell proliferation, the timing of the medium exchange can also be set to a time point when the osmotic pressure exceeds a certain level (for example, 310 m Osm/kg).

One embodiment to which the method of the present invention can be preferably applied includes high density culture using a bioreactor or the like. Specifically, when high density culture is performed using a bioreactor or the like, the pH of the medium may decrease in a relatively short period of time due to the vital activity of a large number of cells. The bioreactor detects a decrease in pH and automatically adds a pH adjuster (e.g., sodium hydrogen carbonate) to the medium to maintain the pH near neutral. Therefore, the osmotic pressure of the medium increases with time. As shown in the following Examples, when the osmotic pressure of the medium exceeds a certain level, the cell proliferation efficiency decreases. Therefore, by timely exchanging a part or whole amount of a medium having an increased osmotic pressure with the medium of the present invention, based on the osmotic pressure of the medium, the osmotic pressure of the medium is adjusted to a range suitable for cell proliferation (i.e., 220 to 310 (preferably 240 to 280) mOsmol/kg). As a result, cell proliferation can be achieved with more preferred efficiency than that previously achieved with bioreactors. In consideration of such fact, the method of the present invention can also be rephrased in one aspect as an improved method of the high density culture method using a bioreactor.

In the method of the present invention, the whole amount of the medium being used may be exchanged or a part (e.g., 10%, 20%, 30%, 40%, 50%, 60%, 70%, 80%, 90%, etc. based on the total amount of the medium in use) thereof may be exchanged at the time of exchange with the medium of the present invention. The amount of the medium to be exchanged is not particularly limited as long as the osmotic pressure after the medium exchange can be within a range suitable for cell culture (that is, 220 to 310 (preferably 240 to 280) mOsmol/kg). However, exchanging the whole amount of medium may cause stress on the cells due to a sudden change in the culture environment. Therefore, in one embodiment, the amount of a medium to be exchanged with the medium of the present invention may be a part of the medium. Specifically, it may be 10% to 90% of the whole amount of a medium in use, preferably 50% to 90% of the whole amount of a medium in use, further preferably 70% to 90% of the whole amount of a medium in use.

In one preferred embodiment, D-glucose and five kinds of amino acids (tryptophan, serine, cysteine (or cystine), methionine, arginine) may be further added to the medium of the present invention.

Glucose (or a salt thereof) can be added to the medium of the present invention such that the converted glucose concentration is generally 0.1 g/L/day to 900 g/L/day, preferably 1 g/L/day to 200 g/L/day, more preferably 1 g/L/day to 20 g/L/day.

In addition, five kinds of amino acids (tryptophan, serine, cysteine (cystine), methionine, and arginine) can be added to the medium of the present invention such that the concentration of tryptophan (concentration after conversion to tryptophan in a free form) is generally 0.1 mg/L/day to 11000 mg/L/day, preferably 1 mg/L/day to 1000 mg/L/day, more preferably 1 mg/L/day to 100 mg/L/day, the concentration of serine (concentration after conversion to serine in a free form) is generally 0.1 mg/L/day to 425000 mg/L/day, preferably 1 mg/L/day to 1000 mg/L/day, more preferably 1 mg/L/day to 100 mg/L/day, the concentration of cysteine or cystine (concentration after conversion to cysteine in a free form) is generally 0.1 mg/L/day to 280000 mg/L/day, preferably 1 mg/L/day to 1000 mg/L/day, more preferably 1 mg/L/day to 100 mg/L/day, the concentration of methionine (concentration after conversion to methionine in a free form) is generally 0.1 mg/L/day to 55000 mg/L/day, preferably 1 mg/L/day to 1000 mg/L/day, more preferably 1 mg/L/day to 100 mg/L/day, and the concentration of arginine (concentration after conversion to arginine in a free form) is generally 0.1 mg/L/day to 150000 mg/L/day, preferably 1 mg/L/day to 2000 mg/L/day, more preferably 1 mg/L/day to 200 mg/L/day.

When the method of the present invention is applied to stem cells, in one preferred embodiment, bFGF and/or choline may be further added to the medium of the present invention.

In the method of the present invention, culture conditions are not particularly limited, and a method known per se may be selected according to the cell type, cell density, culture method (adhesion culture/suspension culture, etc.) and the like. For example, the culture temperature may be generally 25°C to 39°C, preferably 33°C to 39°C. The carbon dioxide concentration may be generally 4% by volume to 10% by volume, preferably 4% by volume to 6% by volume. The oxygen concentration may be generally 1% by volume to 25% by volume, preferably 4% by volume to 20% by volume.

The present invention is explained in more detail in the following Examples; however, the present invention is not limited by these Examples.

### [Example]

In the following Examples, the effect of a medium with reduced osmotic pressure on the proliferation of induced pluripotent stem cells (iPS cells) was evaluated. As the iPS cell, 1210B2 strain purchased from iPS Academia Japan was used. In addition, as a medium for iPS cells, a commercially available StemFit AK03N (Ajinomoto Co., Inc.) was used.

### [Example 1] iPS cell proliferation promoting effect by medium with reduced osmotic pressure in suspension culture system

Using a 30 mL single use bioreactor for iPS cells (ABLE: model number BWV-S03A), iPS cell 1210B2 strain was seeded in StemFit AK03N+10 µM Y-27632 (Wako: 034-24024) at a cell density of 6×10⁵ cells/mL and the cells were cultured with stirring in a CO₂ incubator under the conditions of 37°C, CO₂ concentration=5%, stirring speed=120 rpm. On the second day of seeding, 70% of the medium was replaced with StemFit AK03N. Liquid A of StemFit AK03N was diluted with water for injection (Otsuka Pharmaceutical Co., Ltd.) to prepare StemFit AK03N adjusted to 220 mOsmol/kg. In addition, Liquid A of StemFit AK03N was diluted with water for injection and NaCl (Wako Pure Chemical Industries, Ltd.) was added to prepare StemFit AK03N adjusted to 260 mOsmol/kg. On the third day of seeding, the cell suspension (10 mL) was resuspended in fresh 220 mOsmol/kg StemFit AK03N or 260 mOsmol/kg StemFit AK03N. The cells were transferred to a bioreactor ambr15 (sartorius: 001-0881), and stirring culture was continued under the conditions of 37°C, pH=7.2, dissolved oxygen concentration=20%, stirring speed=300 rpm. 70% of the medium was exchanged once a day with 220 mOsmol/kg StemFit AK03N or 260 mOsmol/kg StemFit AK03N, and 40 mg/L/day Trp (Ajinomoto Co., Inc.), 40 mg/L/day Ser (Ajinomoto Co., Inc.), 40 mg/L/day Cys (Japan protein), 40 mg/L/day Met (Ajinomoto Co., Inc.), 160 mg/L/day Arg (Ajinomoto Co., Inc.), 4 g/L/day D-glucose (Nacalai Tesque:16806-25) were further added to both groups. On the 12th day of culture, the number of viable cells was measured using Vi-CELL^{™} XR (Beckman Coulter), a live/dead cell autoanalyzer. In addition, the osmotic pressure of the culture supernatant on the 12th day of culture was measured using OSMOMAT auto (Gonotec), which is an osmometer for the freezing point depression method.

The verification results of the influence of osmotic pressure on the proliferation of iPS cells in n=1 are shown in Fig. 1, and the osmotic pressure at that time is shown in Fig. 2. Results showing a cell proliferation promoting effect were obtained by adjusting the osmotic pressure during culture with a medium with osmotic pressure adjusted to 220 mOsmol/kg.

### [Example 2] iPS cell proliferation promoting effect by medium with reduced osmotic pressure in suspension culture system

NaCl in StemFit AK03N Liquid A was adjusted to prepare StemFit AK03N having an osmotic pressure of 220, 241, 267, 307, or 352 mOsm/kg. Using a 30 mL single use bioreactor for iPS cells (ABLE: BWV-S03A), iPS cell 1210B2 strain was seeded in StemFit AK03N+10 µM Y-27632 (Wako: 034-24024) at each osmotic pressure at a cell density of 2.6×10⁵ cells/mL and the cells were cultured with stirring in a CO₂ incubator under the conditions of 37°C, CO₂ concentration=5%, stirring speed=120 rpm. After the second day of seeding, 70% of the medium was replaced once a day with StemFit AK03N at each osmotic pressure. On the 4th or 5th day of culture, the number of viable cells was measured using Vi-CELL^{™} XR (Beckman Coulter), a live/dead cell autoanalyzer.

The verification results of the influence of osmotic pressure on the proliferation of iPS cells in n=1 are shown in Fig. 3. Results showing a cell proliferation promoting effect were obtained by adjusting the osmotic pressure of the medium.

### [Industrial Applicability]

According to the present invention, more preferable high density culture as compared with the embodiments performed previously in cell culture using a bioreactor can be realized by a highly inexpensive and simple means.

This application is based on a patent application No. 2020-003960 filed in Japan (filing date: January 14, 2020), the contents of which are incorporated in full herein.

## Claims

1. A medium with a reduced osmotic pressure comprising a basal medium, wherein the osmotic pressure is adjusted to 150 to 250 mOsm/kg.

2. The medium according to claim 1, wherein the osmotic pressure of the medium is 170 to 230 mOsm/kg.

3. The medium according to claim 1 or 2, wherein the osmotic pressure is adjusted by reducing an amount of sodium chloride among the components constituting the basal medium.

4. A method for culturing a cell, comprising a step of exchanging a part or whole amount of a medium being used with the medium according to any one of claims 1 to 3 so that the osmotic pressure of the medium being used is 220 to 310 mOsm/kg.

5. The method according to claim 4, wherein the cell culture is suspension culture.
